# EUROPEAN PATENT SPECIFICATION

(11) **EP 2 878 252 B1**
(45) Date of publication and mention of the grant of the patent: **16.08.2017**
(21) Application number: 14193977.7
(22) Date of filing: 20.11.2014
(51) Int. Cl.: A61B 1/00, A61B 1/015

(54) **Switching valve unit and endoscope apparatus**
Schaltventileinheit und Endoskopvorrichtung
Unité de soupape de commutation et appareil d'endoscope

(30) Priority: 28.11.2013 JP 2013246691
(43) Date of publication of application: 03.06.2015
(73) Proprietor: Fujifilm Corporation, Minato-ku Tokyo 106-8620 (JP)
(72) Inventor: Fukushima Kimitake, Kanagawa (JP); Shihota, Yuji, Kanagawa (JP)
(74) Representative: Klunker IP Patentanwälte PartG mbB

(56) References cited:
- EP-A1- 2 095 757
- EP-A1- 2 441 377
- JP-A- 2007 111 266

## Description

### BACKGROUND OF THE INVENTION

### 1. Field of the Invention

The present invention relates to a switching valve unit according to the preamble of claim 1 and an endoscope apparatus which makes use of such switching valve unit. More particularly, the present invention relates to a switching valve unit and endoscope apparatus, in which a piston rod can be manipulated smoothly by reducing force of depression of the piston rod.

### 2. Description Related to the Prior Art

Ultrasonic imaging is well-known in the field of medical diagnosis. Ultrasonic waves are applied to a body, from which reflected ultrasonic waves are received to form an image for medical diagnosis of an object in a body. An example of the ultrasonic imaging is endoscopic ultrasonography. In comparison with ultrasonic echo imaging of application of the ultrasonic waves from the outside of the body, it is possible precisely to image body tissue of an inner wall of the body cavity, for example, stomach and large intestine. The endoscopic ultrasonography is typically important for diagnosis of depth of a lesion in the inner wall of the body cavity, for example, tumor, ulcer and the like.

Medical instruments for the endoscopic ultrasonography include an ultrasonic endoscope apparatus and an ultrasonic probe. The ultrasonic endoscope apparatus has a tip device with an ultrasonic transducer array and a CCD image sensor. The ultrasonic probe is introduced through a forceps channel or instrument channel in an endoscope apparatus for use in the imaging. Assuming that air is present in a body cavity to which the ultrasonic waves are applied by the ultrasonic endoscope apparatus or the ultrasonic probe, a problem arises in the ultrasonic waves may attenuate considerably. In view of this,
an elastic balloon is disposed on the tip device of the ultrasonic endoscope apparatus or the ultrasonic probe for covering the ultrasonic transducer array. The balloon is inflated by supplying water or ultrasonic transmission medium, and set in contact with a wall of the body cavity, or for anchoring. Then the ultrasonic waves are emitted from the inside of the balloon. Thus, attenuation of the ultrasonic waves due to air is prevented. In case the ultrasonic imaging is terminated, the water is drawn out of the balloon. The ultrasonic endoscope apparatus can be pulled out and removed from the body by deflating the balloon.

Inflation and deflation of the balloon are changed over by manipulating a fluid button unit and/or a suction button unit disposed on a control handle (handle device) of the endoscope apparatus. A widely used example of the suction button unit or the endoscope apparatus is a button unit of the two-step structure (switching valve unit), as disclosed in U.S. Pat. Pub. 2012/088,975 (corresponding to JP-B 5250601), JP-A 2005-058547 (corresponding to JP-B 4394394), JP-A 2007-014439 (corresponding to JP-B 4619217), and JP-A 10-028670 (corresponding to JP-B 3017957).

The fluid button unit of U.S. Pat. Pub. 2012/088,975 has a button cap and a vent formed in the button cap. In case an air pump is driven, air is leaked through the vent. In case the vent is closed, a check valve disposed in the fluid button unit is opened, so that a fluid nozzle ejects the air. In case the button cap is depressed halfway, the fluid button unit is changed over from the air supply to water supply, for the fluid nozzle to eject water. In case the button cap is depressed fully, the channels are changed over. A delivery conduit for balloon inflation upstream of the balloon is caused to supply water to inflate the balloon.

While the suction button unit of U. S. Pat. Pub. 2012/088,975 is free without depression, air is drawn from the atmosphere by a suction pump through a passageway. In case the button cap in the suction button unit is halfway depressed, the suction pump comes to communicate with the instrument channel for suction. In case the button cap in the suction button unit is fully depressed, the suction pump comes to communicate with the delivery conduit. The balloon is deflated by discharging water from the balloon through the delivery conduit.

The suction button unit, as a button unit of the two-step structure, includes a valve cylinder, a piston rod, the button cap and a cylinder cap (cap device). The piston rod is contained in the valve cylinder movably, and has a piston head protruding from a piston opening. The button cap is disposed on the piston head. The cylinder cap is disposed to cover the piston opening, and keeps the button cap operable for halfway depression and full depression.

Various conduits are connected to an inner chamber of the valve cylinder, including a discharge conduit (35), an exhaust conduit (connection tube) and a suction conduit. The discharge conduit is disposed near to the piston opening and communicates with the balloon. The exhaust conduit is disposed at a proximal end from the discharge conduit and communicates with the suction pump. The suction conduit is disposed at a lower end of the valve cylinder and communicates with the instrument channel. A valve head of the piston rod has an inner chamber for communication between its lateral surface and a tip surface of the valve head. A recess or groove is formed in a lateral surface of the valve head. In case the button cap is depressed halfway, the chamber, recess and the like connects the exhaust conduit with the suction conduit. In case the button cap is depressed fully, the chamber, recess and the like connects the exhaust conduit with the discharge conduit.

In case the balloon is advanced into a body cavity of a body together with a tip device of the ultrasonic endoscope apparatus, force of backflow of water in the balloon occurs because of pressure from the body cavity. In U.S. Pat. Pub. 2012/088,975, the ultrasonic endoscope apparatus has seal packing, such as an O-ring, disposed on the button cap near to the discharge conduit so as to prevent leakage of water from the discharge conduit in an inactive state or halfway depressed state. Also, the endoscope apparatus in JP-A 2005-058547 and JP-A 2007-014439 has the seal packing for closing the discharge conduit in an inactive state or halfway depressed state. The endoscope apparatus in JP-A 10-028670 has a passage, disposed at a lower end of the valve cylinder, for communication with the suction conduit and the discharge conduit. Two pistons are slid up and down with respect to the passage. In case the button cap is depressed halfway, the exhaust conduit communicates with the suction conduit. In case the button cap is depressed fully, the exhaust conduit communicates with the discharge conduit. The seal packing is provided on each of the pistons for sealing in a fluid-tight manner inside the valve cylinder.

However, the endoscope apparatus of any one of those patent documents has plural pieces of the seal packing, so that friction between the seal packing and the valve cylinder is considerably high in the course of the depression. The button unit cannot be manipulated smoothly due to large force of depression. Also, the suction conduit must be washed with a washing brush or the like in order to eliminate waste fluid or body fluid from the body cavity. However, such a washing brush cannot easily reach the suction conduit in the endoscope apparatus of those patent documents, because the suction conduit is disposed behind the valve cylinder. Washability of the suction conduit is low.

In accordance with the preamble of claim 1, JP 2007-111266 discloses a switching valve unit which has two piston rods, i.e. a suction piston and a balloon suction piston.

### SUMMARY OF THE INVENTION

In view of the foregoing problems, an object of the present invention is to provide a switching valve unit and endoscope apparatus, in which a piston rod can be manipulated smoothly by reducing force of depression of the piston rod.

In order to achieve the above and other objects and advantages of this invention, a switching valve unit as defined by claim 1 having a piston rod, movable in an axial direction, for changing over plural conduits, is provided. A valve cylinder has a piston chamber, for containing the piston rod, and keeping the piston rod slidable between a first position on an upper side, a second position lower than the first position in the axial direction, and a halfway position between the first and second positions. A receiving opening is defined in an upper end of the piston chamber. A discharge port hole is defined in a lower end of the piston chamber, for communicating with a discharge conduit. A suction port hole is formed in the valve cylinder, disposed near to the receiving opening, for communicating with a suction conduit. An exhaust port hole is formed in the valve cylinder, disposed nearer to the suction port hole than the discharge port hole, for communicating with an exhaust conduit. Seal packing is disposed on the piston rod on a side of the exhaust port hole, for keeping the piston rod fluid-tight in relation to a wall of the piston chamber. A relief passage is formed in the wall of the piston chamber, has a larger width than the seal packing, for receiving the seal packing in case the piston rod is in the second position. A first passage is formed in the piston rod, for externally opening the exhaust port hole in case the piston rod is in the first position. A second passage is formed in the piston rod, for connecting the suction port hole with the exhaust port hole in case the piston rod is in the halfway position. A third passage connects the discharge port hole with the exhaust port hole by use of the relief passage in case the piston rod is in the second
position. A cap device associates the piston rod with the valve cylinder, and sets the piston rod in the first and second positions and the halfway position.

Preferably, the discharge conduit and the suction conduit are formed through an elongated tube of an endoscope apparatus, and the valve cylinder is disposed on a control handle of the endoscope apparatus.

Preferably, furthermore, a first anti-rotation device regulates relative rotation between the piston rod and the cap device. A second anti-rotation device regulates relative rotation between the cap device and the valve cylinder.

Preferably, the piston chamber includes a tapered surface disposed with an inclination toward the receiving opening. The suction port hole is disposed in an externally observable manner from the tapered surface.

Preferably, the second anti-rotation device includes an engagement projection formed on a first one of the tapered surface and the cap device. An engagement recess is formed in a second one of the tapered surface and the cap device, for receiving the engagement projection for engagement.

Preferably, the piston rod includes a flow path surface, formed in a peripheral surface longer than a stroke between the first and second positions in the axial direction, having a portion of positioning the exhaust port hole while positioned in the first and second positions, for alignment with a space in the tapered surface while positioned in the first position, to constitute the first passage.

Preferably, the piston rod includes a large diameter portion disposed near to the upper end of the piston chamber. A small diameter portion is formed under the large diameter portion, has a smaller diameter than the larger diameter portion, and has
the seal packing, for enabling fluid to flow inside the piston chamber. The piston chamber includes a piston slide surface for guiding the large diameter portion in a slidable manner. A sliding sealing surface is disposed under the piston slide surface, for keeping the seal packing slidable and fluid-tight.

Preferably, the third passage includes a first flow path surface, formed in a peripheral surface of the large diameter portion to extend in the axial direction, and aligned with the exhaust port hole. A second flow path surface is formed in the peripheral surface of the large diameter portion at a phase angle different from the first flow path surface, to correspond to a lower portion of the first flow path surface with reference to the axial direction, and opposed to the piston slide surface. A passage hole is formed through the large diameter portion, to open through each of the first and second flow path surfaces. In case the piston rod is in the second position, the first flow path surface, the passage hole and the second flow path surface open a flow path between the exhaust port hole and the relief passage.

Preferably, the endoscope apparatus further includes a balloon, disposed on a tip device of the elongated tube, connected with an end of the discharge conduit, for anchoring in the body cavity.

Also, an endoscope apparatus is provided, and includes a control handle, an elongated tube, disposed to extend from the control handle longitudinally, for imaging an object in a body cavity, and a balloon, disposed on a tip device of the elongated tube, for anchoring in the body cavity. A discharge conduit is formed through the elongated tube, for connection to the balloon. A suction conduit is formed through the elongated tube, and has a suction opening positioned at the tip device of the elongated tube. An exhaust conduit is disposed to extend from the control handle toward a proximal side, for connection to a suction source. The endoscope apparatus includes the switching valve unit as claimed. A suction port hole is formed in the valve cylinder, disposed near to the receiving opening, for communicating with the suction conduit. An exhaust port hole is formed in the valve cylinder, disposed nearer to the suction port hole than the discharge port hole, for communicating with an exhaust conduit. Seal packing is disposed on the piston rod on a side of the exhaust port hole, for keeping the piston rod fluid-tight in relation to a wall of the piston chamber. A relief passage is formed in the wall of the piston chamber, has a larger width than the seal packing, for receiving the seal packing in case the piston rod is in the second position. A first passage is formed in the piston rod, for externally opening the exhaust port hole in case the piston rod is in the first position. A second passage is formed in the piston rod, for connecting the suction port hole with the exhaust port hole in case the piston rod is in the halfway position. A third passage connects the discharge port hole with the exhaust port hole by use of the relief passage in case the piston rod is in the second position.

Consequently, the piston rod can be manipulated smoothly by reducing force of depression of the piston rod, because the structure with the seal packing is sufficiently simple.

### BRIEF DESCRIPTION OF THE DRAWINGS

The above objects and advantages of the present invention will become more apparent from the following detailed description when read in connection with the accompanying drawings, in which:
Fig. 1 is an explanatory view illustrating an endoscope apparatus;
Fig. 2 is a perspective view illustrating a suction button unit mounted on a control handle;
Fig. 3 is a vertical section illustrating the suction button unit before depression;
Fig. 4 is a perspective view illustrating a cap device (cylinder cap) and a piston rod;
Fig. 5 is an exploded perspective view illustrating the piston rod;
Fig. 6 is a perspective view illustrating the cap device;
Fig. 7 is a bottom perspective view illustrating the cap device;
Fig. 8 is a cross section taken on line VIII-VIII in Fig. 3;
Fig. 9 is a vertical section illustrating the suction button unit in a state of regulating rotation;
Fig. 10 is a vertical section illustrating the suction button unit at the time of halfway depression;
Fig. 11 is a vertical section illustrating the suction button unit at the time of full depression.

### DETAILED DESCRIPTION OF THE PREFERRED EMBODIMENT(S) OF THE PRESENT INVENTION

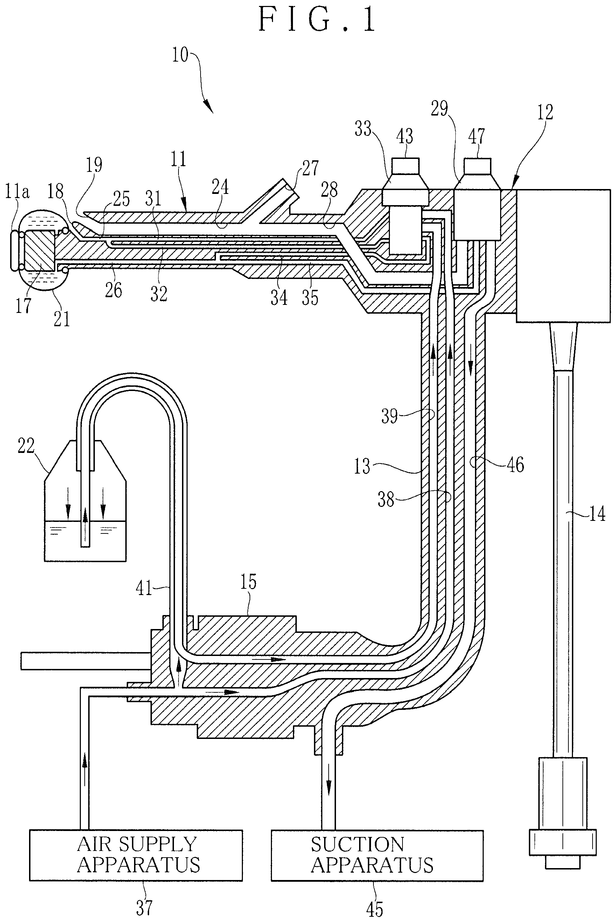

In Fig. 1, an ultrasonic endoscope apparatus 10 includes an elongated tube 11 or guide tube, a control handle 12 (handle device), a universal cable 13 (connection tube) and a connection cable 14. The elongated tube 11 is advanced for entry in a body cavity of a patient, for example, gastrointestinal tract. The control handle 12 is disposed at a proximal end of the elongated tube 11. First ends of the universal cable 13 and the connection cable 14 are disposed at the control handle 12. A connector plug 15 is disposed at a second end of the universal cable 13, and used for connection with a processing apparatus, light source apparatus (all not shown) and the like. The connection cable 14 is used for connection with a processing apparatus for endoscopic ultrasonography.

The elongated tube 11 is flexible in a rod form of a cylindrical shape. A tip device 11a of the elongated tube 11 includes an ultrasonic transducer array 17, a CCD image sensor (not shown), a fluid nozzle 18 and a distal opening 19 (suction opening). The ultrasonic transducer array 17 for endoscopic ultrasonography transmits and receives ultrasonic waves and forms an ultrasonic image. The image sensor optically detects image light to form an endoscopic image. The fluid nozzle 18 washes a viewing window (not shown) for imaging. The distal opening 19 is an outlet of an instrument such as forceps, and also a suction opening for drawing fluid, such as blood, body fluid and the like.

An elastic balloon 21 is secured to the tip device 11a in a removable manner. At first, the balloon 21 is deflated and fitted on the outside of the tip device 11a before advance into the body cavity. A water tank 22 or water supply source supplies the balloon 21 with water for inflation while the ultrasonic transducer array 17 emits ultrasonic waves. The balloon 21 operates for anchoring the tip device 11a on a cavity wall of the body cavity. Also, the balloon 21 prevents attenuation of ultrasonic waves and reflected ultrasonic waves in the presence of air upon emission from the ultrasonic transducer array 17. After the inflation, the balloon 21 is deflated by discharge of water. An example of the material of the balloon 21 is latex rubber.

Plural channels are formed to extend through the elongated tube 11 and the control handle 12, including an instrument channel 24, a fluid channel 25 and a balloon channel 26 (or tube lumens) . The instrument channel 24 has a distal end with the distal opening 19. The fluid channel 25 has a distal end with the fluid nozzle 18. A distal end of the balloon channel 26 communicates with an inner space of the balloon 21. In Fig. 1, portions other than the instrument channel 24, the fluid channel 25 and the balloon channel 26 are hatched for distinction in their depiction.

A proximal instrument opening 27 is formed in the elongated tube 11 at a proximal end of the instrument channel 24. While the proximal instrument opening 27 is not used for entry of a medical instrument, a closure device (not shown) closes the proximal instrument opening 27. A suction conduit 28 is a branch of the instrument channel 24. A suction button unit 29 or switching valve unit of the present invention is disposed on the control handle 12. The suction conduit 28 extends to the suction button unit 29.

A second end of the fluid channel 25 has two branches including an air channel 31 and a water channel 32 (or tube lumens) . A fluid button unit 33 is disposed on the control handle 12, and is connected with the air channel 31 and the water channel 32. A second end of the balloon channel 26 has two branches including a delivery conduit 34 for balloon inflation, and a discharge conduit 35 for balloon deflation. The delivery conduit 34 communicates to the fluid button unit 33. The discharge conduit 35 communicates to the suction button unit 29.

Various conduits are coupled to the fluid button unit 33, including the air channel 31, the water channel 32, the delivery conduit 34, an air supply conduit 38 and a water supply conduit 39. An air supply apparatus 37 or air supply source is connected by the air supply conduit 38 to the fluid button unit 33. The water tank 22 is connected by the water supply conduit 39 to the fluid button unit 33. During the endoscopic ultrasonography, the air supply apparatus 37 is always active.

A branch conduit 41 extends from a point of a rear end portion of the air supply conduit 38 within the connector plug 15. The branch conduit 41 is connected to the water tank 22. A portion of a rear end of the water supply conduit 39 is extended through the branch conduit 41 to the inside of the water tank 22. An inner pressure of the water tank 22 is increased by supply of air from the air supply apparatus 37 through the branch conduit 41, to draw water from the water tank 22 to the water supply conduit 39.

The fluid button unit 33 is a two-way button and also three-way valve. A button cap 43 of the fluid button unit 33 has a vent hole (not shown) open to the atmosphere. While the button cap 43 is not depressed, the fluid button unit 33 closes the water supply conduit 39, and causes the air supply conduit 38 to communicate with the vent hole of the button cap 43. Thus, air from the air supply conduit 38 is leaked through the vent hole in the fluid button unit 33. Furthermore, closing the vent hole causes communication of the air supply conduit 38 with the air channel 31 in a state of closing the water supply conduit 39, in the manner disclosed in U.S. Pat. Pub. 2012/088,975 (corresponding to JP-B 5250601). The air is drawn to the air channel 31 and ejected from the fluid nozzle 18.

Upon depressing the button cap 43 halfway, the fluid button unit 33 closes the air supply conduit 38 and connects the water supply conduit 39 only with the water channel 32. Water drawn by the water supply conduit 39 flows through the water channel 32 and is ejected from the fluid nozzle 18. Upon depressing the button cap 43 fully, the fluid button unit 33 keeps the air supply conduit 38 closed, and connects the water supply conduit 39 only with the delivery conduit 34. Thus, water drawn by the water supply conduit 39 flows through the delivery conduit 34 toward the inside of the balloon 21.

There is an exhaust conduit 46 or exhaust conduit tube (suction source conduit or suction source conduit tube) having first and second ends. The first end of the exhaust conduit 46 is coupled to a suction apparatus 45 or suction source. The second end of the exhaust conduit 46 is coupled to the suction button unit 29 in addition to the suction conduit 28 and the discharge conduit 35. The suction apparatus 45 is also active during the endoscopic ultrasonography. The suction button unit 29 is a two-way button and also three-way valve similar to the fluid button unit 33.

While a button cap 47 of the suction button unit 29 is not manipulated, the suction button unit 29 opens the exhaust conduit 46 6 to the atmosphere. This is because load to the suction apparatus 45 may increase should the exhaust conduit 46 be closed from the atmosphere in a constantly active condition of the suction apparatus 45. It is possible to prevent an increase of load to the suction apparatus 45 by opening the exhaust conduit 46 to the atmosphere.

In case the button cap 47 is depressed halfway, the suction button unit 29 causes the exhaust conduit 46 to communicate only with the suction conduit 28. Thus, negative pressure or suction force in the suction conduit 28 and the instrument channel 24 is increased to draw and remove body fluid or the like through the distal opening 19. Upon fully depressing the button cap 47, the suction button unit 29 causes the exhaust conduit 46 to communicate only with the discharge conduit 35. Negative pressure or suction force in the discharge conduit 35 and the balloon channel 26 is increased to discharge water from the balloon 21.

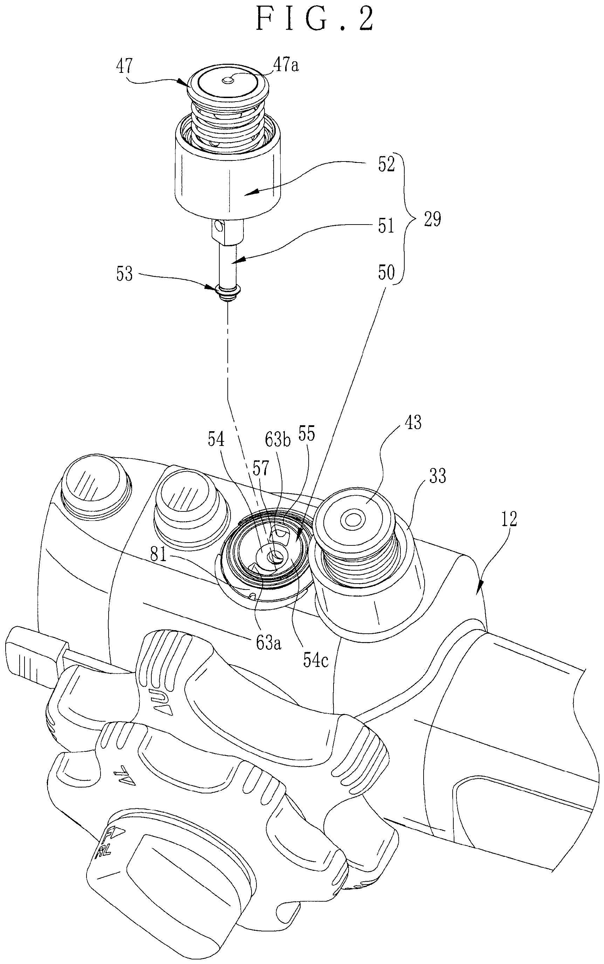

In Fig. 2, the suction button unit 29 includes a valve cylinder 50, a piston rod 51 or valve stem, a cap device 52 or cylinder cap, and seal packing 53. The valve cylinder 50 is mounted on the control handle 12 fixedly. The piston rod 51 is contained in the valve cylinder 50 in a slidable manner. The cap device 52 is disposed on the valve cylinder 50, and sets the piston rod 51 in a first position, halfway position and second position. The piston rod 51 in the first position which is higher than the halfway position is free from depression. The seal packing 53 is attached to the piston rod 51. In Fig. 2, the piston rod 51, the cap device 52 and the seal packing 53 are separated from the valve cylinder 50 for the purpose of clarification in the disclosure.

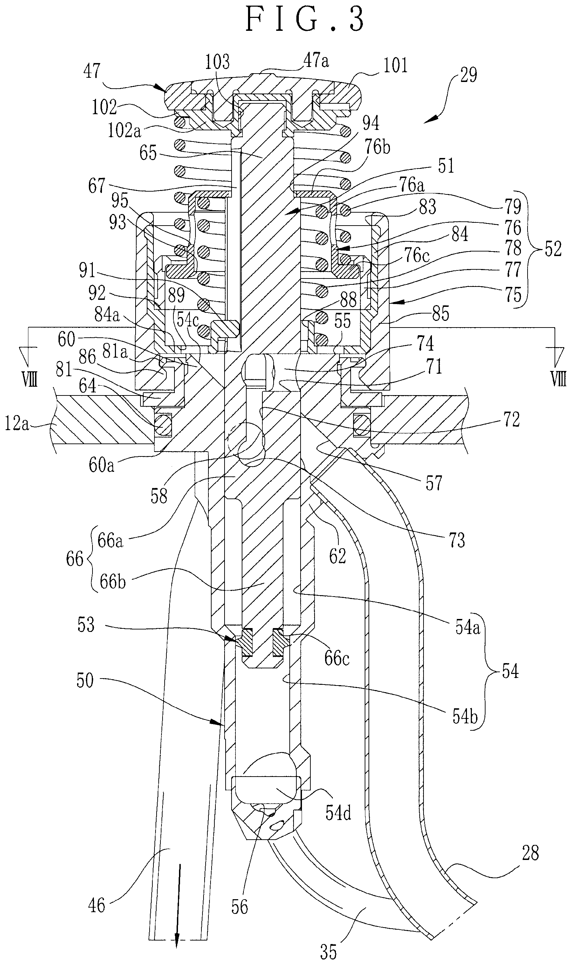

In Fig. 3, the valve cylinder 50 is a barrel of metal having two bores. A piston chamber 54 or valve chamber is defined in the valve cylinder 50. The piston chamber 54 has a piston slide surface 54a and a sliding sealing surface 54b. The piston slide surface 54a has a large diameter. The sliding sealing surface 54b has a smaller diameter than the piston slide surface 54a. A receiving opening 55 is formed to open at an upper end of the piston chamber 54. A discharge port hole 56 is formed at a lower end of the piston chamber 54. The discharge conduit 35 is coupled to the discharge port hole 56.

A suction port hole 57, and an exhaust port hole 58 or suction source port hole are formed in the piston slide surface 54a and arranged near to the receiving opening 55 with an interval in a circumferential direction. The suction conduit 28 is coupled to the suction port hole 57. The exhaust conduit 46 is coupled to the exhaust port hole 58.

A support sleeve 60 is formed on the valve cylinder 50 at its upper end, for receiving contact of a lower surface of the cap device 52. A tapered surface 54c is formed with the piston slide surface 54a having the support sleeve 60, and is tapered in an upward direction toward the receiving opening 55.

The suction port hole 57 is formed to communicate through the piston slide surface 54a so that an axis of the suction port hole 57 is parallel with the tapered surface 54c and that the suction port hole 57 is externally observable in a downward direction along the tapered surface 54c. Thus, the suction port hole 57 is positioned to appear through the receiving opening 55. A suction connector 62 is disposed between connection ends of the suction port hole 57 and the suction conduit 28. The suction conduit 28 is set in and secured to the suction connector 62.

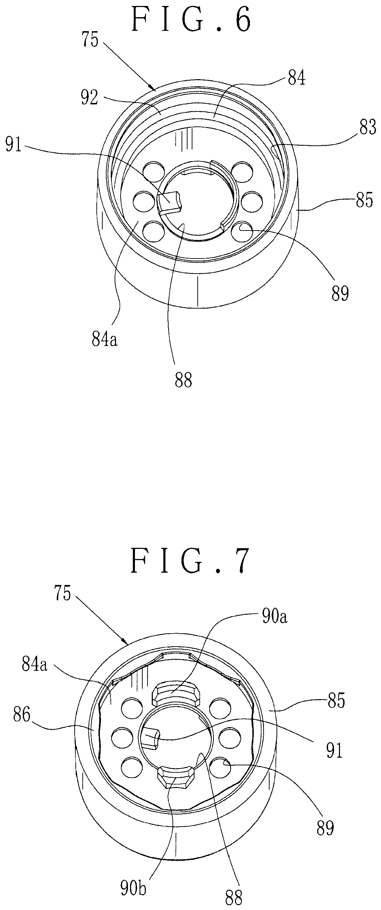

In Fig. 2, engagement recesses 63a and 63b in an anti-rotation device for the cap device 52 are formed in the tapered surface 54c and arranged at an interval of 180 degrees. Engagement projections 90a and 90b in the anti-rotation device for the cap device 52 in Fig. 7 are engaged with the engagement recesses 63a and 63b, for coupling the valve cylinder 50 with the cap device 52 firmly without rotation. A male thread (not shown) is formed about the support sleeve 60 at its upper end. There is a mount ring 81 having a female thread, as will be described later. The male thread is helically engaged with the female thread.

In Fig. 3, a coupling flange 60a is formed on a lower surface of the support sleeve 60. A lower surface of a handle housing 12a of the control handle 12 contacts the coupling flange 60a. The valve cylinder 50 is fastened to the handle housing 12a by helical engagement of the mount ring 81 with the male thread of the support sleeve 60. Seal packing 64 or sealing is fitted around the coupling flange 60a to enclose a space with the handle housing 12a in a fluid-tight manner.

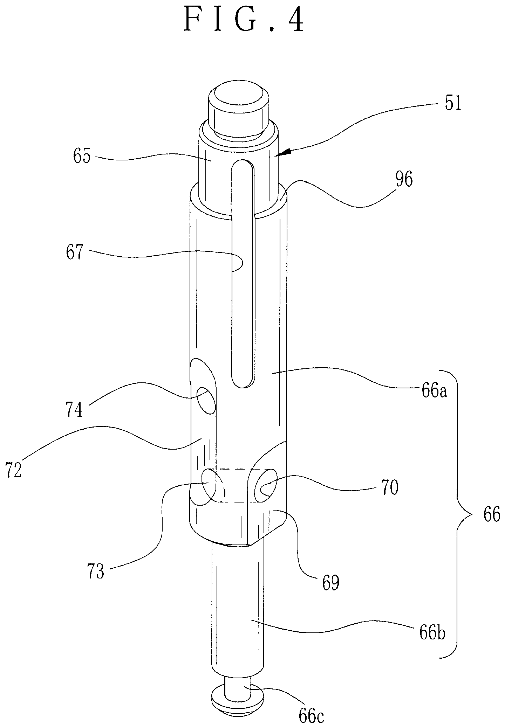

In Fig. 4, the piston rod 51 is a rod of metal, and includes a piston head 65 and a valve shaft 66 in a downward direction. The valve shaft 66 includes a large diameter portion 66a and a small diameter portion 66b (or valve head), and entered in the piston chamber 54 of Fig. 2. In Fig. 3, the large diameter portion 66a is slid inside the piston slide surface 54a. A ring groove 66c is formed in the small diameter portion 66b and near to its lower end. The seal packing 53 of elastic material is fitted in the ring groove 66c.

The small diameter portion 66b has an outer diameter smaller than an inner diameter of the sliding sealing surface 54b, and is movable in the sliding sealing surface 54b with a space. The seal packing 53 in the ring groove 66c has an outer diameter equal to or slightly larger than an inner diameter of the sliding sealing surface 54b, to seal an interface between the small diameter portion 66b and the sliding sealing surface 54b in a fluid-tight manner. A form of the seal packing 53 is annular and has a projection of which a section is triangular. However, various forms of the seal packing 53 can be used for tightly closing the small diameter portion 66b and the sliding sealing surface 54b, for example, an O-ring as a commercially available product. A relief passage 54d is formed with the sliding sealing surface 54b, is adjacent with the discharge port hole 56 and has an outer diameter larger than that of the seal packing 53. In the operation of the full depression, the seal packing 53 enters the relief passage 54d to open the flow path, for communication from the sliding sealing surface 54b to the discharge port hole 56.

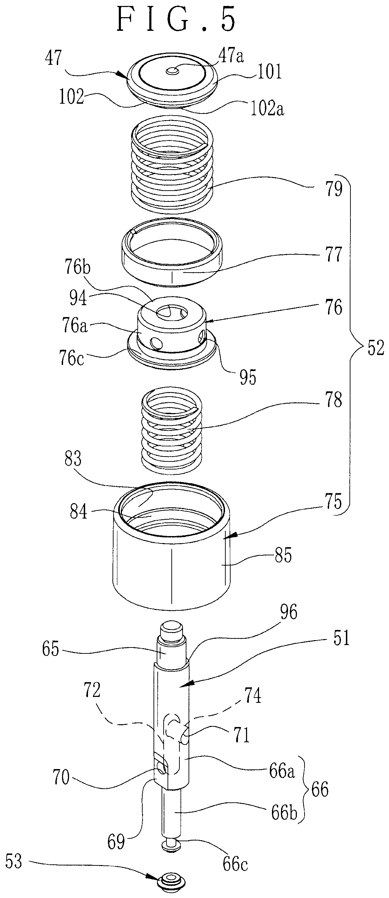

The button cap 47 is mounted fixedly on the tip of the piston head 65 and depressible manually for halfway depression and full depression. In Fig. 5, a button indicia 47a is formed on an upper surface of the button cap 47 for indicating a position of manual touch for the depression.

The large diameter portion 66a includes a lower flow path surface 69, a lower hole opening 70, an upper hole opening 71, a flow path surface 72, a lower passage hole 73 and an upper passage hole 74. The flow path surface 69 (corresponding to a second flow path surface) is formed by chamfering a shoulder portion adjacent to the small diameter portion 66b. The lower hole opening 70 is formed through a wall of the flow path surface 69. The upper hole opening 71 is disposed higher than the lower hole opening 70. The flow path surface 72 (corresponding to a first flow path surface) is formed by chamfering a peripheral surface to extend in the axial direction. The lower passage hole 73 is a through hole from the lower hole opening 70 to the flow path surface 72. The upper passage hole 74 is a through hole from the upper hole opening 71 to the flow path surface 72. The upper and lower hole openings 70 and 71 and the flow path surface 72 are arranged at different phase angles in a circumferential direction of the large diameter portion 66a.

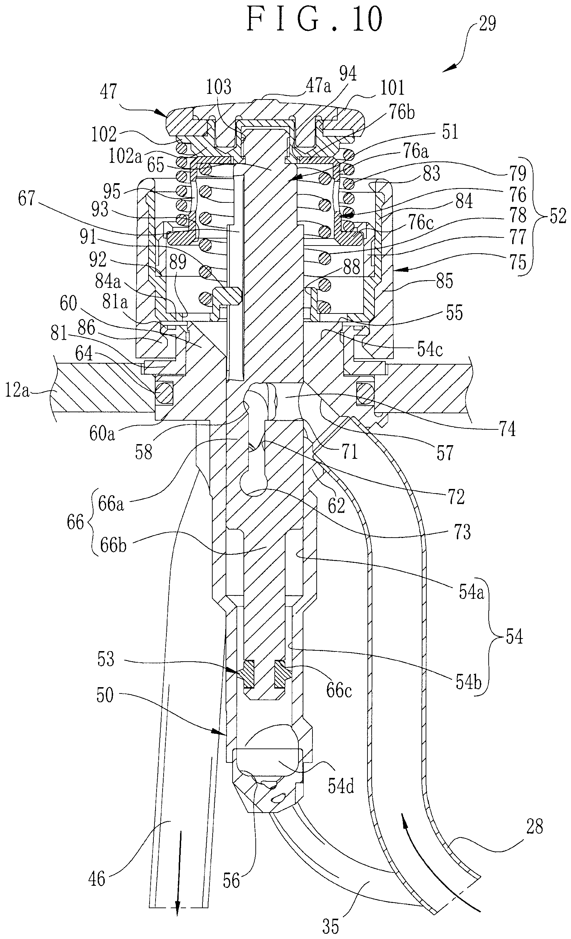

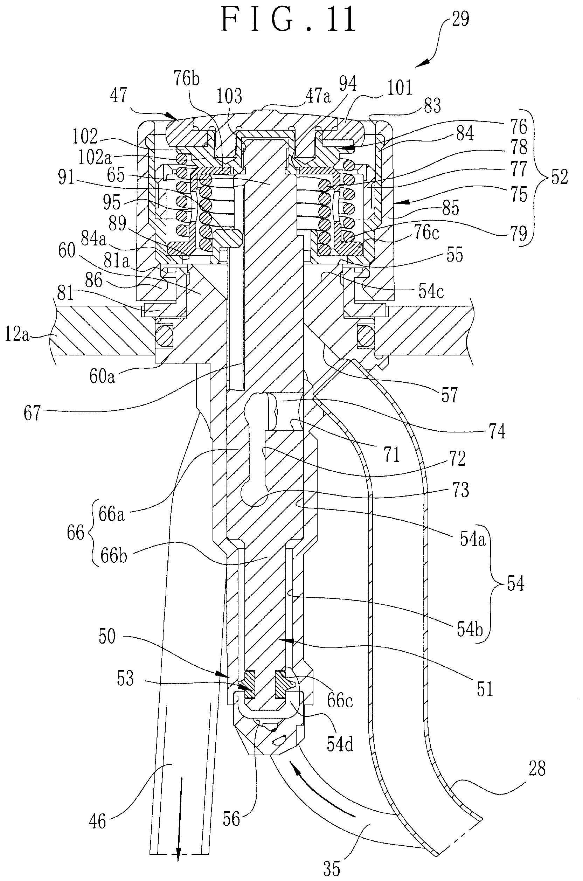

The upper hole opening 71 is positioned with the tapered surface 54c in Fig. 3 in the first position, but becomes aligned with the suction port hole 57 in the halfway position in Fig. 10, and becomes positioned with the piston slide surface 54a in the second position in Fig. 11. The flow path surface 72 is always aligned with the exhaust port hole 58. A length of the flow path surface 72 is larger in the axial direction than a stroke of the piston rod 51 from the first position to the second position. The flow path surface 72 in the first position is aligned with a space in the tapered surface 54c.

In Fig. 4, an engagement groove 67 (key groove) in an anti-rotation device for the piston rod is formed in the piston head 65 and the large diameter portion 66a, extends in an axial direction, and is used for blocking rotation with the cap device 52. A length of the engagement groove 67 is larger than a slidable range of the piston rod 51 in the axial direction for the purpose of safely sliding the piston rod 51 without interference.

The piston rod 51 is kept slidable between a first position (inactive position) and a second position (fully depressed position) by positioning with the cap device 52. In Fig. 3, the piston rod 51 is in the first position. The button cap 47 is not depressed but is the most distant from the receiving opening 55. In Fig. 11, the piston rod 51 is in the second position. The button cap 47 is set the nearest to the receiving opening 55 by the full depression and prevented from further depression. Furthermore, the piston rod 51 is set in a halfway position (third position) between the first and second positions by the cap device 52.

In Fig. 3, the piston rod 51 is in the first position, so that the exhaust port hole 58, the suction port hole 57 and the discharge port hole 56 are closed from communication with one another by the seal packing 53 and an outer surface of the valve shaft 66. A flow space of the flow path surface 72 operates as a first passage and becomes aligned with the exhaust port hole 58. Also, the flow path surface 72 becomes aligned with a partial space in the tapered surface 54c to open the exhaust port hole 58 to the atmosphere.

In Fig. 10, the piston rod 51 comes to the halfway position upon the halfway depression of the button cap 47. The flow space of the flow path surface 72 cooperates with the upper passage hole 74 as a second passage, for connecting the exhaust port hole 58 with the suction port hole 57 for suction.

In Fig. 11, the piston rod 51 is in the second position. The seal packing 53 enters the relief passage 54d. A third passage becomes constituted by a gap between the sliding sealing surface 54b, the relief passage 54d and the small diameter portion 66b, and the flow path surface 69, the lower passage hole 73 and the flow space of the flow path surface 72. The discharge port hole 56 is connected with the exhaust port hole 58 in a condition of the balloon deflation.

The cap device 52 includes a button housing 75, an inner cap 76, an inner support ring 77, a first compression coil spring 78 and a second compression coil spring 79. The button housing 75 has a lower plate portion, and is disposed at the upper end of the valve cylinder 50 and around the receiving opening 55. The button housing 75, the inner cap 76 and the inner support ring 77 of the cap device 52 have larger diameters than that of the upper end of the valve cylinder 50.

The button housing 75 is mounted on an end of the valve cylinder 50 with the mount ring 81 of Fig. 3. A hole is defined in the mount ring 81 for receiving entry of the support sleeve 60. A female thread (not shown) is formed with an inner surface of the hole. A male thread of the support sleeve 60 is helically engaged with the female thread of the mount ring 81, to couple the mount ring 81 to the tip of the valve cylinder 50. Also, an annular flange 81a is formed with an upper portion of the mount ring 81.

In Fig. 6, the button housing 75 includes an intermediate sleeve 84 of metal, and a cover sleeve 85 of resin. The intermediate sleeve 84 has a sleeve opening 83 open at its top. The cover sleeve 85 is fitted on an outer surface of the intermediate sleeve 84 for covering. In Fig. 7, a lower end portion of the cover sleeve 85 extends toward the valve cylinder 50 lower than a lower surface of the intermediate sleeve 84. In Fig. 4, plural coupling claws 86 are formed on an inner surface of the lower end portion for engagement with the annular flange 81a. In Fig. 3, the engagement of the coupling claws 86 with the annular flange 81a keeps the intermediate sleeve 84 positioned on the valve cylinder 50 in a removable manner by cooperation of the cover sleeve 85 and the mount ring 81.

A lower plate 84a of the intermediate sleeve 84 contacts an upper surface of the support sleeve 60. A center hole 88 or cylinder hole is formed in the lower plate 84a for receiving entry of the piston head 65 of the piston rod 51. A plurality of wall vents 89 are formed in the lower plate 84a and opposed to the tapered surface 54c. Thus, a flow path from the inside of the intermediate sleeve 84 to the upper passage hole 74 in the valve cylinder 50 is opened for flow of air in the inactive state before the depression.

In Fig. 7, the engagement projections 90a and 90b are formed on the lower plate 84a. In Fig. 2, the engagement projections 90a and 90b are entered in respectively the engagement recesses 63a and 63b in the valve cylinder 50 for blocking rotation. The engagement projection 90b is different from the engagement projection 90a in the width. The engagement projection 90a is engageable with the engagement recess 63a, while the engagement projection 90b is engageable with the engagement recess 63b. Note that other anti-rotation devices can be provided in place of the engagement projections 90a and 90b and the engagement recesses 63a and 63b. For example, an engagement recess may be formed in the cap device 52. An engagement projection may be formed on the valve cylinder 50, and may be engageable with the engagement recess for blocking rotation between the valve cylinder 50 and the cap device 52.

In Fig. 7, an engagement projection 91 (key projection) in the anti-rotation device for the piston rod is formed on an inner surface of the center hole 88 to project toward the axis radially. The engagement projection 91 is engaged with the engagement groove 67 of the piston rod 51 for blocking rotation. Note that other anti-rotation structures can be used, for example, an engagement projection on the piston rod 51 and an engagement recess in the cap device 52. In Fig. 6, an inner annular shoulder 92 is formed with the intermediate sleeve 84 by forming an annular step on an inner wall of the sleeve opening 83.

In Fig. 3, the inner cap 76 is kept slidable on an inner surface of the intermediate sleeve 84, and prevented from dropping out of the intermediate sleeve 84 by the inner support ring 77 mounted on the inner annular shoulder 92. The inner cap 76 includes a cap plate 76b, a cap skirt 76a and an annular flange 76c. The cap skirt 76a extends in the axial direction of the piston rod 51 and downwards from the cap plate 76b. The annular flange 76c is formed on an outer surface of the cap skirt 76a at its lower end. There is a cap opening 93 defined in an upper end of the cap skirt 76a. Diameters of the cap skirt 76a and the cap opening 93 are predetermined larger than an inner diameter of the receiving opening 55 and smaller than an inner diameter of the intermediate sleeve 84.

A receiving hole 94 is formed through the cap plate 76b for receiving entry of the piston head 65. A plurality of cap vents 95 are formed in the cap skirt 76a. The cap vents 95 are open to the atmosphere. Thus, the exhaust conduit 46 is opened to the atmosphere by the flow path surface 72, the upper passage hole 74, the wall vents 89, the inside of the intermediate sleeve 84 and the inner cap 76, and the cap vents 95. See Fig. 2.

An annular shoulder 96 or anti-drop portion is formed on the valve shaft 66 as illustrated in Fig. 5. As an inner diameter of the receiving hole 94 is smaller than a diameter of the valve shaft 66, the cap plate 76b contacts the annular shoulder 96. Thus, the piston rod 51 is kept positioned on the valve cylinder 50 without drop by the inner cap 76, the inner support ring 77, the button housing 75 and the mount ring 81.

The inner cap 76 is slidable between an upper position (protrusion position) and a lower position (containment position). Upon being set in the upper position, the annular flange 76c of the inner cap 76 contacts the inner support ring 77 to set the cap plate 76b higher than the sleeve opening 83. See Figs. 3 and 10. Upon being set in the lower position, a lower end of the cap skirt 76a of the inner cap 76 contacts the lower plate 84a to set the cap plate 76b lower than the sleeve opening 83. See Fig. 11. The inner cap 76 is in the upper position while the piston rod 51 is moved from the first position to the halfway position.

While the piston rod 51 is moved from the halfway position to the second position by the push of the button cap 47, the inner cap 76 is moved by the button cap 47 from the upper position to the lower position. Upon movement of the piston rod 51 to the second position, the inner cap 76 moves to the lower position, and prevents the button cap 47 and the piston rod 51 from moving down further.

The first compression coil spring 78 is disposed between the lower plate 84a and the cap plate 76b in a state compressed in the axial direction of the piston rod 51 more shortly than its free length. The first compression coil spring 78 biases the cap plate 76b in a direction to protrude from the sleeve opening 83 to maintain the inner cap 76 in the upper position.

The second compression coil spring 79 is disposed between the annular flange 76c and the button cap 47 in a state compressed in the axial direction of the piston rod 51 more shortly than its free length. The piston rod 51 is disposed through the second compression coil spring 79. The second compression coil spring 79 biases the button cap 47 in a direction protruding from the receiving hole 94. The second compression coil spring 79 is so prepared that its force of bias is smaller than that of the first compression coil spring 78. In the course of the depression of the button cap 47, at first the second compression coil spring 79 starts being deformed, and then the first compression coil spring 78 starts being deformed. It is possible to set the button cap 47 stopped in the halfway position temporarily by utilizing a difference in the force of the bias between the compression coil springs 78 and 79.

The compression coil springs 78 and 79 are disposed in the cap device 52 together with the inner cap 76. The second compression coil spring 79 is positioned outside the inner cap 76 in contrast with the first compression coil spring 78 inside the inner cap 76, in a form of a double structure. Thus, a height of the cap device 52 can be set small by the tight containment of the compression coil springs 78 and 79 in the cap device 52.

The piston rod 51 is kept in the inactive position by the compression coil springs 78 and 79. It is necessary to depress the button cap 47 against the bias of the second compression coil spring 79 in order to move the piston rod 51 from the inactive position to the halfway position. Also, it is necessary to depress the button cap 47 against the bias of the compression coil springs 78 and 79 in order to move the piston rod 51 from the halfway position to the second position. The total force of the bias applied to the button cap 47 is changed in a path of the piston rod 51 from the first position to the second position.

The button cap 47 includes a cap disk 101 of resin, and a pressure plate 102 of metal. The pressure plate 102 is mounted on a lower surface of the cap disk 101 fixedly, and is pressed on the cap plate 76b while the piston rod 51 is set in a range from the halfway position to the second position. A disk wall 102a is formed under the pressure plate 102, and projects toward the cap plate 76b. A screw hole 103 is formed in the disk wall 102a, and has a female thread (not shown). A male thread (not shown) is formed around the piston head 65, and helically engaged with the screw hole 103 to couple the pressure plate 102 to the piston head 65. While the piston rod 51 is in the second position, a lower end of the disk wall 102a is pressed on the cap plate 76b, and the cap skirt 76a is pressed on the lower plate 84a.

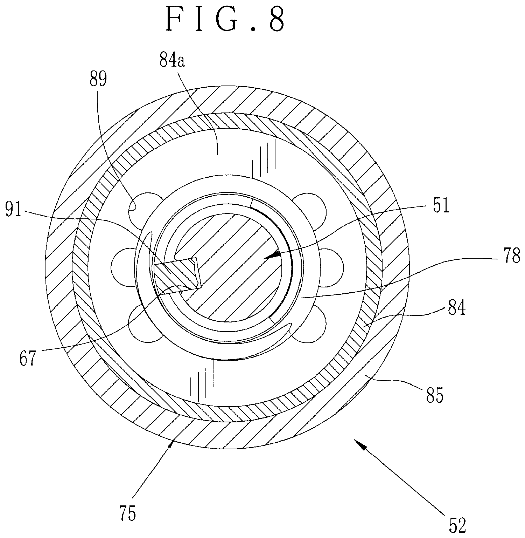

In Fig. 8 taken on line VIII-VIII in Fig. 3, the piston rod 51 is prevented from rotating in the button housing 75 by engagement between the engagement projection 91 (key projection) on the lower plate 84a and the engagement groove 67 (key groove) in the piston rod 51. As the button housing 75 is fixedly mounted in the valve cylinder 50, the piston rod 51 can be kept from rotating about the axis in the valve cylinder 50 without a direct anti-rotation structure.

The engagement projections 90a and 90b formed on the intermediate sleeve 84 enter respectively the engagement recesses 63a and 63b of the valve cylinder 50 for engagement, to block rotation between the valve cylinder 50 and the button housing 75. The coupling claws 86 of the cover sleeve 85 are coupled with the annular flange 81a of the mount ring 81 to mount the button housing 75 in the valve cylinder 50 firmly in addition to the engagement between the engagement recesses 63a and 63b and the engagement projections 90a and 90b.

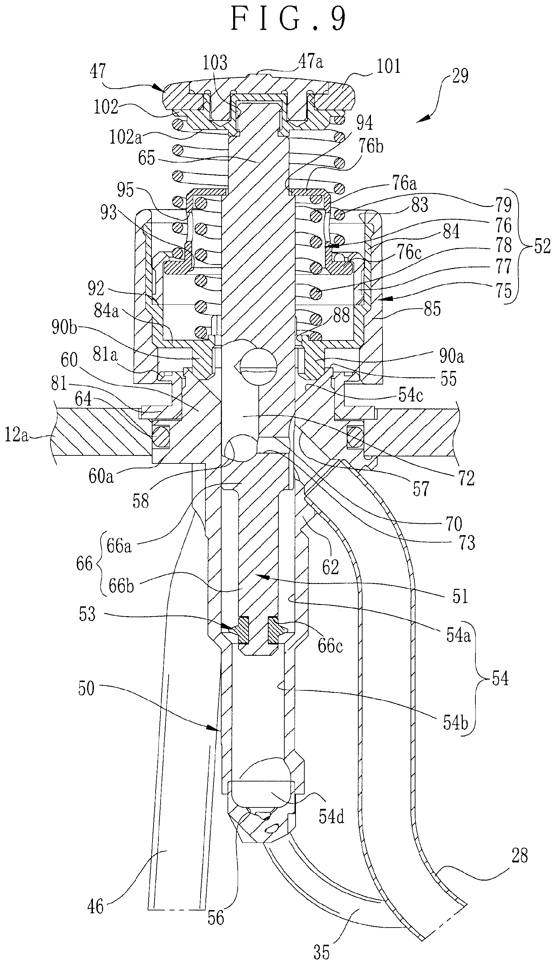

In Fig. 9, the valve shaft 66 of the piston rod 51 is contained in the piston chamber 54. Assuming that the engagement groove 67 is disengaged from the engagement projection 91, the seal packing 53 is disposed on a higher side than the sliding sealing surface 54b, as the engagement projections 90a and 90b contact the tapered surface 54c. Then the piston rod 51 is fixedly rotated together with the cap device 52 from the state of Fig. 9 (in a clockwise direction according to Fig. 5) and depressed. It is possible even with such a simple manipulation to engage the engagement groove 67 with the engagement projection 91. See Fig. 3. In the engaged state, the seal packing 53 is movable in contact with the sliding sealing surface 54b. It is possible in the assembly of the piston rod 51 and the valve cylinder 50 to contain the piston rod 51 in the piston chamber 54 and set the seal packing 53 in contact with the sliding sealing surface 54b.

To remove the piston rod 51 and the cap device 52 from the valve cylinder 50 for the purpose of washing the ultrasonic endoscope apparatus 10, the button housing 75 of the cap device 52 is pinched and rotated manually. The engagement projections 90a and 90b of Fig. 7 are shifted from the engagement recesses 63a and 63b of the valve cylinder 50, to disengage the coupling claws 86 from the annular flange 81a as illustrated in Fig. 9. In Fig. 2, the piston rod 51 and the cap device 52 are separated from the valve cylinder 50. Note that ends of the engagement projections 90a and 90b in the circumferential direction are tapered with an inclination, so that their removal from the engagement recesses 63a and 63b is facilitated.

The operation of the suction button unit 29 in the ultrasonic endoscope apparatus 10 is described now. At the time of readiness for endoscopic ultrasonography, the CCD image sensor and the ultrasonic transducer array 17 are driven. The air supply apparatus 37 and the suction apparatus 45 are continuously actuated for air supply and suction. Then the elongated tube 11 is entered in a body cavity of a body of a patient, for example, gastrointestinal tract, to start imaging of an object of interest. The balloon 21 is deflated by discharge of water and compressed in tight contact with an outer surface of the tip device 11a.

For diagnosis of the gastrointestinal tract, at first an endoscopic image from the CCD is viewed. The button cap 43 of the fluid button unit 33 is manipulated for ejecting air or fluid through the fluid nozzle 18 according to requirement of washing an object of interest or washing a viewing window (not shown) of the tip device 11a. The imaging is changed over to endoscopic ultrasonography for detailed imaging, for example, for a discovered lesion in the body cavity after the first imaging with the endoscopic image.

For the endoscopic ultrasonography, the button cap 43 is depressed fully to supply water from the water tank 22 to the balloon 21 through the water supply conduit 39, the delivery conduit 34 and the balloon channel 26, to inflate the balloon 21. In the water supply to the balloon 21, a well-known adjusting method for an amount of the water is used. Then the balloon 21 is positioned and anchored on body tissue with a lesion or other object of interest in the body cavity to be imaged. An ultrasonic image of the object is acquired.

In the normal imaging without suction or balloon deflation, the suction button unit 29 of the button cap 47 is not depressed in Fig. 3 during the endoscopic ultrasonography or endoscopic imaging. The piston rod 51 is maintained in the first position to close the flow path by the compression coil springs 78 and 79. The flow path surface 72 is aligned with the exhaust port hole 58. However, the lower passage hole 73 and the upper passage hole 74 are not aligned with respectively the discharge port hole 56 and the suction port hole 57. The suction conduit 28 and the discharge conduit 35 are closed from communication with the exhaust conduit 46 by tight sealing of the seal packing 53 on the sliding sealing surface 54b. No suction through the distal opening 19 occurs. No discharge of water from the balloon 21 occurs for deflation.

While the piston rod 51 is in the first position, the exhaust port hole 58 is opened to the atmosphere by the flow path surface 72, the lower passage hole 73, the upper passage hole 74, the tapered surface 54c, the wall vents 89 and the cap vents 95. As a result, occurrence of load to the suction apparatus 45 can be prevented even while there is no suction from the distal opening 19 or no discharge of water from the balloon 21.

Assuming that it is necessary to suck and remove fluid, such as blood, body fluid or the like in the course of endoscopic ultrasonography or endoscopic imaging, the button cap 47 is depressed halfway to depress the piston rod 51 in the receiving opening 55. Force of bias of the second compression coil spring 79 is applied to the button cap 47 until the piston rod 51 moves to the halfway position. Also, force of bias of the compression coil springs 78 and 79 is applied to the button cap 47 after the piston rod 51 moves past the halfway position. It is possible to stop the piston rod 51 in the halfway position, because the force of the bias applied to the button cap 47 is increased according to the halfway position.

In Fig. 10, the piston rod 51 is changed over from the inactive state to the suction upon positioning in the halfway position. The upper hole opening 71 becomes aligned with the suction port hole 57. The flow path surface 72 is aligned with the exhaust port hole 58. As the seal packing 53 tightly contacts the sliding sealing surface 54b tightly, the suction port hole 57 comes to communicate with the exhaust port hole 58.

Upon the connection of the exhaust port hole 58 with the suction port hole 57, the exhaust conduit 46 comes to communicate with the suction conduit 28 and the instrument channel 24 through the flow path surface 72 and the upper passage hole 74. Thus, fluid of various types can be drawn through the distal opening 19. The fluid is moved out to the outside of the ultrasonic endoscope apparatus 10 through the instrument channel 24, the suction conduit 28, the piston chamber 54, the upper passage hole 74, the flow space of the flow path surface 72 and the exhaust conduit 46.

In the first position and halfway position, the balloon 21 receives pressure from the body cavity. Backflow of water may occur through the discharge conduit 35. However, no water flows through the suction conduit 28 and the exhaust conduit 46 because the seal packing 53 contacts the sliding sealing surface 54b in a fluid-tight manner. In case stop of the suction is desired, the button cap 47 is left from manual push. The bias of the second compression coil spring 79 returns the piston rod 51 to the first position of Fig. 3.

At the end of the endoscopic ultrasonography, the button cap 47 is depressed fully to push the piston rod 51 in the receiving opening 55. Force of bias from the second compression coil spring 79 is applied to the button cap 47 before the piston rod 51 moves past the halfway position. However, the force of bias from the compression coil springs 78 and 79 is applied to the button cap 47 after the piston rod 51 moves past the halfway position. Also, the depression of the button cap 47 moves the inner cap 76 from the upper position to the lower position. In the course of continuing the push of the button cap 47 against the bias of the compression coil springs 78 and 79, the inner cap 76 reaches the lower position and kept from further depression. Thus, the piston rod 51 is stopped in the full depressed position.

In Fig. 11, the piston rod 51 changes over to the balloon deflation by stopping in the second position. The upper hole opening 71 comes out of alignment with the suction port hole 57 in the axial direction of the piston rod 51. The flow path surface 72 is aligned with the exhaust port hole 58. Also, the seal packing 53 enters the relief passage 54d to open the flow path, so that the discharge port hole 56 comes to communicate with the exhaust port hole 58.

Upon the connection of the exhaust port hole 58 with the discharge port hole 56, the exhaust conduit 46 comes to communicate with the discharge conduit 35 and the balloon channel 26 through the gap between the relief passage 54d and the small diameter portion 66b, the sliding sealing surface 54b, the flow path surface 69, the lower passage hole 73 and the flow path surface 72. Thus, negative pressure or force of suction increases in the flow path from the exhaust conduit 46 to the balloon channel 26. The balloon 21 is deflated by discharge of the water. The water from the balloon 21 is drained to the outside of the ultrasonic endoscope apparatus 10 through the balloon channel 26, the discharge conduit 35, the gap between the relief passage 54d and the small diameter portion 66b, the sliding sealing surface 54b, the flow space of the flow path surface 69, the lower passage hole 73, the flow space of the flow path surface 72 and the exhaust conduit 46.

Then discharge of water of a predetermined amount from the balloon 21 is detected by use of a publicly known method of detecting an amount of discharged water. A physician or operator leaves his or her finger from the button cap 47 to release the button cap 47. The force of bias of the compression coil springs 78 and 79 returns the piston rod 51 to the first position of Fig. 3.

Similarly, the fluid and suction button units 33 and 29 are suitably manipulated until the end of the imaging of the ultrasonic endoscope apparatus 10. The steps of the air supply, water supply, balloon inflation, suction and balloon deflation are performed.

In the present embodiment, the discharge port hole 56 is disposed at the lower end in the piston chamber 54. The suction port hole 57 is near to the receiving opening 55 in the piston chamber 54. The exhaust port hole 58 is disposed lower than the suction port hole 57. The seal packing 53 as a single product operates for sealing in the states of the first position and halfway position, to block flow of water from the discharge conduit 35. The use of only the seal packing 53 makes frictional force small in the slide of the suction button unit 29. It is possible to manipulate the suction button unit 29 only with small force for smoothing the manipulation. Also, the suction port hole 57 is near to the receiving opening 55. It is possible to wash the suction conduit 28 with high washability, because a washing brush into the suction conduit 28 can be used even with possibility in deposition of body fluid or unwanted fluid. Furthermore, the flow path surface 72 and the upper and lower passage holes 73 and 74 in the piston rod 51 are in a constant condition of receiving negative pressure in communication with the exhaust conduit 46. It is possible to prevent leakage of water, body fluid or the like even in case the piston rod 51 is returned to the first position from the halfway position or the second position.

In the above embodiment, the tapered surface 54c is formed with the piston chamber 54. However, the tapered surface 54c can be a surface other than a conical surface. For example, tapered lines of a cross section of the tapered surface 54c may be curved convexly or concavely, so that the tapered surface 54c can be a parabolic surface or a spherical surface. In the above embodiments, the ultrasonic endoscope apparatus 10 has the suction button unit 29. However, an endoscope apparatus having the suction button unit 29 according to the invention can be any one of various endoscope apparatuses, for example, a colonoscope as an endoscope apparatus for a large intestine.

In the suction apparatus 45 or suction source, any suitable examples of negative pressure sources can be used, for example, fan, blower, compressor, pump or the like. The suction apparatus 45 has a collection tank (not shown). Water from the balloon or body fluid from the body cavity is withdrawn by the negative pressure source and collected in the collection tank.

In the above embodiment, the suction button unit 29 is so disposed as to orient the button cap 47 on an upper side. The terms of the upper and lower sides are used according to the direction of depressing the suction button unit 29. However, the suction button unit 29 of the invention can be disposed to orient the button cap 47 on a lateral side, lower side or the like other than the upper side.

In the above embodiment, the flow path surfaces 69 and 72 are flat surfaces formed by chamfering the peripheral surface of the large diameter portion 66a of the valve shaft 66. However, the flow path surfaces 69 and 72 can be formed by cutting out the large diameter portion 66a at a suitable depth. A flow space of the flow path surfaces 69 and 72 can be constituted by a passage groove.

In the above embodiment, the switching valve unit is used for the endoscope apparatus. However, a switching valve unit of the invention can be used for a probe, catheter, or other apparatuses having conduits, for medical use or for diagnosis.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A switching valve unit (29), comprising:
a piston rod (51), movable in an axial direction;
a valve cylinder (50), having a piston chamber (54), for containing said piston rod, and keeping said piston rod slidable between a first position on an upper side, a second position lower than said first position in said axial direction, and a halfway position between said first and second positions;
a receiving opening (55) defined in an upper end of said piston chamber;
a discharge port hole (56), defined in a lower end of said piston chamber, for communicating with a discharge conduit (35);
a suction port hole (57), formed in said valve cylinder, disposed near to said receiving opening, for communicating with a suction conduit (28);
an exhaust port hole (58), formed in said valve cylinder, disposed nearer to said suction port hole than said discharge port hole, for communicating with an exhaust conduit (46);
seal packing (53), disposed on said piston rod on a side of said exhaust port hole, for keeping said piston rod fluid-tight in relation to a wall of said piston chamber;
a relief passage (54d), formed in said wall of said piston chamber, having a larger width than said seal packing, for receiving said seal packing in case said piston rod is in said second position;
a first passage (72);
a second passage (72, 74), formed in said piston rod, for connecting said suction port hole with said exhaust port hole in case said piston rod is in said halfway position;
a third passage (69, 72, 73) for connecting said discharge port hole with said exhaust port hole by use of said relief passage in case said piston rod is in said second position, and
a cap device (52);
**characterized in that** said piston rod (51) is a single piston rod (51) adapted to change over between said suction conduit (28), said discharge conduit (35) and said exhaust conduit (46), that said first passage (72) is formed in said piston rod (51), for externally opening said exhaust port hole (58) in case said piston rod (51) is in said first position and that said cap device (52) is adapted to associate said piston rod (51) with said valve cylinder (50), and to set said piston rod (51) in said first and second positions and said halfway position.

2. A switching valve unit as defined in claim 1, wherein said discharge conduit and said suction conduit are formed through an elongated tube of an endoscope apparatus, and said valve cylinder is disposed on a control handle of said endoscope apparatus.

3. A switching valve unit as defined in claim 1, further comprising:
a first anti-rotation device for regulating relative rotation between said piston rod and said cap device;
a second anti-rotation device for regulating relative rotation between said cap device and said valve cylinder.

4. A switching valve unit as defined in any one of claims 1 to 3, wherein said piston chamber includes a tapered surface disposed with an inclination toward said receiving opening;
said suction port hole is disposed in an externally observable manner from said tapered surface.

5. A switching valve unit as defined in claim 3 or in claim 4, wherein said second anti-rotation device includes:
an engagement projection formed on a first one of said tapered surface and said cap device;
an engagement recess, formed in a second one of said tapered surface and said cap device, for receiving said engagement projection for engagement.

6. A switching valve unit as defined in claim 4, wherein said piston rod includes a flow path surface, formed in a peripheral surface longer than a stroke between said first and second positions in said axial direction, having a portion of positioning said exhaust port hole while positioned in said first and second positions, for alignment with a space in said tapered surface while positioned in said first position, to constitute said first passage.

7. A switching valve unit as defined in any one of claims 1 to 6, wherein said piston rod includes:
a large diameter portion disposed near to said upper end of said piston chamber;
a small diameter portion, formed under said large diameter portion, having a smaller diameter than said larger diameter portion, and having said seal packing, for enabling fluid to flow inside said piston chamber;
said piston chamber includes:
a piston slide surface for guiding said large diameter portion in a slidable manner;
a sliding sealing surface, disposed under said piston slide surface, for keeping said seal packing slidable and fluid-tight.

8. A switching valve unit as defined in claim 7, wherein said third passage includes:
a first flow path surface, formed in a peripheral surface of said large diameter portion to extend in said axial direction, and aligned with said exhaust port hole;
a second flow path surface, formed in said peripheral surface of said large diameter portion at a phase angle different from said first flow path surface, to correspond to a lower portion of said first flow path surface with reference to said axial direction, and opposed to said piston slide surface;
a passage hole, formed through said large diameter portion, to open through each of said first and second flow path surfaces;
in case said piston rod is in said second position, said first flow path surface, said passage hole and said second flow path surface open a flow path between said exhaust port hole and said relief passage.

9. A switching valve unit as defined in any one of claims 2 to 8, wherein said endoscope apparatus further includes a balloon, disposed on a tip device of said elongated tube, connected with an end of said discharge conduit, for anchoring in said body cavity.

10. An endoscope apparatus including a control handle (12), an elongated tube (11), disposed to extend from said control handle longitudinally, for imaging an object in a body cavity, and a balloon (21), disposed on a tip device of said elongated tube, for anchoring in said body cavity, said endoscope apparatus comprising:
a discharge conduit (35), formed through said elongated tube, for connection to said balloon;
a suction conduit (28), formed through said elongated tube, and having a suction opening positioned at said tip device of said elongated tube;
an exhaust conduit (46), disposed to extend from said control handle toward a proximal side, for connection to a suction source (45); and
a switching valve unit as defined by any of claims 1 to 9,
wherein said discharge port hole (56)
communicates with said discharge conduit (35);
said suction port hole (57) communicates with said suction conduit (28); and
said exhaust port hole (58) communicates with said exhaust conduit (46).

11. The switching valve unit as defined in any one of the preceding claims, further comprising a button cap (47) for manipulation mounted fixedly on a tip of the piston rod (51).

## Patentansprüche

1. Umschaltventileinheit (29), umfassend:
eine Kolbenstange (51), die in axialer Richtung beweglich ist;
einen Ventilzylinder (50) mit einer Kolbenkammer (54) zur Aufnahme der Kolbenstange und zum Halten der Kolbenstange verschieblich zwischen einer ersten Position auf einer Oberseite, einer zweiten Position tiefer als die erste Position in der axialen Richtung, und einer Halbposition zwischen der ersten und der zweiten Position;
eine Aufnahmeöffnung (55), die in einem oberen Ende der Kolbenkammer gebildet ist;
ein Austragöffnungsloch (56), ausgebildet in einem unteren Ende der Kolbenkammer, um mit einer Austragleitung (35) zu kommunizieren;
ein Saugöffnungsloch (57), gebildet in dem Ventilzylinder in der Nähe der Aufnahmeöffnung, um mit einer Saugleitung (28) zu kommunizieren;
ein Ausleitöffnungsloch (58), gebildet in dem Ventilzylinder, näher an dem Ansaugöffnungsloch gelegen als das Austragöffnungsloch, um mit einer Ausleitleitung (46) zu kommunizieren;
eine Dichtungspackung (53), angeordnet an der Kolbenstange auf einer Seite des Ausleitöffnungslochs, um die Kolbenstange fluiddicht in Bezug auf eine Wand der Kolbenkammer zu halten;
einen Entlastungskanal (54d), der in der Wand der Kolbenkammer ausgebildet ist und der eine größere Breite besitzt als die Dichtungspackung, um die Dichtungspackung für den Fall aufzunehmen, dass die Kolbenstange sich in der zweiten Position befindet;
einen ersten Kanal (72);
einen zweiten Kanal (72, 74), ausgebildet in der Kolbenstange zum Verbinden des Ansaugöffnungslochs mit dem Ausleitöffnungsloch für den Fall, dass die Kolbenstange sich in der Halbposition befindet;
einen dritten Kanal (69, 72, 73) zum Verbinden des Austragöffnungslochs mit dem Ausleitöffnungsloch unter Verwendung des Entlastungskanals für den Fall, dass die Kolbenstange sich in der zweiten Position befindet, und
eine Deckeleinrichtung (52);
**dadurch gekennzeichnet, dass** die Kolbenstange (51) eine Einzelkolbenstange (51) ist, ausgebildet zum Wechsel zwischen der Saugleitung (28), der Austragleitung (35) und der Ausleitleitung (46), dass der erste Kanal (72) in der Kolbenstange (51) ausgebildet ist, um das Ausleitöffnungsloch (58) nach außen für den Fall zu öffnen, dass die Kolbenstange (51) sich in der ersten Position befindet, und dass die Deckeleinrichtung (52) dazu ausgebildet ist, die Kolbenstange (51) mit dem Ventilzylinder (50) zusammenzubringen und die Kolbenstange (51) in der ersten und der zweiten Position und der Halbposition einzustellen.

2. Umschaltventileinheit nach Anspruch 1, bei der die Austragleitung und die Saugleitung gebildet sind durch einen länglichen Schlauch einer Endoskopvorrichtung und der Ventilzylinder sich in einem Steuerhandgriff der Endoskopvorrichtung befindet.

3. Umschaltventileinheit nach Anspruch 1, weiterhin umfassend:
eine erste Drehsperreinrichtung zum Regulieren der relativen Drehung zwischen dem Kolben und der Deckeleinrichtung;
eine zweite Drehsperreinrichtung zum Regulieren der relativen Drehung zwischen der Deckeleinrichtung und dem Ventilzylinder.

4. Umschaltventileinheit nach einem der Ansprüche 1 bis 3, bei der die Kolbenkammer eine verjüngte Oberfläche mit einer Neigung in Richtung auf die Aufnahmeöffnung enthält;
das Saugöffnungsloch sich in einer von außen beobachtbaren Weise von der verjüngten Fläche befindet.

5. Umschaltventileinheit nach Anspruch 3 oder Anspruch 4, bei der die zweite Drehsperreinrichtung enthält:
einen Eingriffsvorsprung, der an einem ersten Teil von der verjüngten Oberfläche und der Deckeleinrichtung ausgebildet ist;
eine Eingriffsvertiefung, die an einem zweiten Teil von der verjüngten Fläche und der Deckeleinrichtung ausgebildet ist, um den Eingriffsvorsprung für einen Eingriff aufzunehmen.

6. Umschaltventileinheit nach Anspruch 4, bei der die Kolbenstange eine Strömungswegfläche aufweist, gebildet in einer Umfangsfläche länger als ein Hub zwischen der ersten und der zweiten Position in axialer Richtung, enthaltend einen Abschnitt zum Positionieren des Ausleitöffnungslochs in der ersten und der zweiten Position zwecks Ausrichtung mit einem Raum in der verjüngten Fläche bei Positionierung in der ersten Position, um den ersten Kanal zu bilden.

7. Umschaltventileinheit nach einem der Ansprüche 1 bis 6, bei der die Kolbenstange enthält:
einen Abschnitt großen Durchmessers in der Nähe des oberen Endes der Kolbenkammer;
einen Abschnitt kleinen Durchmessers, gebildet unterhalb des Abschnitts großen Durchmessers, und mit einem kleineren Durchmesser als der Abschnitt großen Durchmessers, und mit der Dichtungspackung, um zu ermöglichen, dass ein Fluid in das Innere der Kolbenkammer strömt;
wobei die Kolbenkammer enthält,
eine Kolbengleitfläche zum Führen des Abschnitts großen Durchmessers in verschieblicher Weise;
eine Gleitdichtungsfläche, angeordnet unterhalb der Kolbengleitfläche, um die Dichtungspackung verschieblich und fluiddicht zu halten.

8. Umschaltventileinheit nach Anspruch 7, bei der der dritte Kanal enthält:
eine erste Strömungswegfläche, gebildet in einer Umfangsfläche des Abschnitts großen Durchmessers, welcher sich in axialer Richtung und ausgerichtet mit dem Ausleitöffnungsloch erstreckt;
eine zweite Strömungswegfläche, gebildet in der Fläche des Abschnitts großen Durchmessers unter einem Phasenwinkel verschieden von der ersten Strömungswegfläche, um einem unteren Bereich der ersten Strömungswegfläche in Bezug auf die axiale Richtung und gegenüber der Kolbengleitfläche zu entsprechen;
ein Kanalloch, welches durch den Abschnitt großen Durchmessers gebildet ist, um sich durch jede von der ersten und der zweiten Strömungswegfläche zu öffnen;
für den Fall, dass die Kolbenstange sich in der zweiten Position befindet, die erste Strömungswegfläche, das Kanalloch und die zweite Strömungswegfläche einen Strömungsweg zwischen dem Ausleitöffnungsloch und dem Entlastungskanal öffnen.

9. Umschaltventileinheit nach einem der Ansprüche 2 bis 8, bei der die Endoskopvorrichtung weiterhin einen Ballon enthält, angeordnet an einem spitzen Teil des länglichen Schlauchs und verbunden mit einem Ende der Austragleitung zwecks Verankerung in dem Körperhohlraum.

10. Endoskopvorrichtung mit einem Steuerhandgriff (12), einem länglichen Schlauch (11), der sich ausgehend von dem Steuerhandgriff in Längsrichtung erstreckt, um ein Objekt in einem Körperhohlraum abzubilden, und einen Ballon (21), der an einem spitzen Teil des länglichen Schlauchs angeordnet ist zur Verankerung in dem Körperhohlraum, wobei die Endoskopvorrichtung enthält:
eine Austragleitung (35), die durch den länglichen Schlauch hindurch zur Verbindung mit dem Ballon ausgebildet ist;
eine Saugleitung (28), die durch den länglichen Schlauch hindurch ausgebildet ist und eine Saugöffnung besitzt, welche sich an dem spitzen Teil des länglichen Schlauchs befindet;
eine Ausleitleitung (46), angeordnet zur Erstreckung ausgehend von dem Steuerhandgriff in Richtung auf eine proximale Seite zwecks Verbindung mit einer Saugquelle (45); und
eine Umschaltventileinheit nach einem der Ansprüche 1 bis 9,
wobei das Austragöffnungsloch (56) mit der Austragleitung (35) kommuniziert;
das Ansaugöffnungsloch (57) mit der Saugleitung (28) kommuniziert; und
das Ausleitöffnungsloch (58) mit der Ausleitleitung (46) kommuniziert.

11. Umschaltventileinheit nach einem der vorhergehenden Ansprüche, weiterhin umfassend einen Knopfdeckel (47) zum Manipulieren, der fest an einem Ende der Kolbenstange (51) angebracht ist.

## Revendications

1. Unité de soupape de commutation (29), comprenant :
une tige de piston (51), mobile dans une direction axiale ;
un cylindre de soupape (50), présentant une chambre de piston (54), pour contenir ladite tige de piston, et maintenir ladite tige de piston d'une manière permettant le coulissement entre une première position sur un côté supérieur, une seconde position inférieure à ladite première position dans ladite direction axiale, et une position de mi-course entre lesdites première et seconde positions ;
une ouverture de réception (55) définie dans une extrémité supérieure de ladite chambre de piston ;
un trou d'orifice de refoulement (56), défini dans une extrémité inférieure de ladite chambre de piston, pour communiquer avec une conduite de refoulement (35) ;
un trou d'orifice d'aspiration (57), formé dans ledit cylindre de soupape, disposé près de ladite ouverture de réception, pour communiquer avec une conduite d'aspiration (28) ;
un trou d'orifice d'échappement (58), formé dans ledit cylindre de soupape, disposé plus près dudit trou d'orifice d'aspiration que dudit trou d'orifice de refoulement, pour communiquer avec une conduite d'échappement (46) ;
une garniture d'étanchéité (53), disposée sur ladite tige de piston sur un côté dudit trou d'orifice d'échappement, pour maintenir ladite tige de piston étanche aux fluides en relation avec une paroi de ladite chambre de piston ;
un passage de surpression (54d), formé dans ladite paroi de ladite chambre de piston, présentant une largeur supérieure à ladite garniture d'étanchéité, pour recevoir ladite garniture d'étanchéité dans le cas où ladite tige de piston se trouve dans ladite seconde position ;
un premier passage (72) ;
un deuxième passage (72, 74), formé dans ladite tige de piston, pour relier ledit trou d'orifice d'aspiration audit trou d'orifice d'échappement dans le cas où ladite tige de piston se trouve dans ladite position de mi-course ;
un troisième passage (69, 72, 73) pour relier ledit trou d'orifice de refoulement audit trou d'orifice d'échappement en utilisant ledit passage de surpression dans le cas où ladite tige de piston se trouve dans ladite seconde position, et
un dispositif de bouchon (52) ;
**caractérisée en ce que** ladite tige de piston (51) est une tige de piston unique (51) apte à permuter entre ladite conduite d'aspiration (28), ladite conduite de refoulement (35) et ladite conduite d'échappement (46), **en ce que** ledit premier passage (72) est formé dans ladite tige de piston (51), pour ouvrir de manière externe ledit trou d'orifice d'échappement (58) dans le cas où ladite tige de piston (51) se trouve dans ladite première position, et **en ce que** ledit dispositif de bouchon (52) est apte à associer ladite tige de piston (51) audit cylindre de soupape (50), et à régler ladite tige de piston (51) dans lesdites première et seconde positions et ladite position de mi-course.

2. Unité de soupape de commutation selon la revendication 1, dans laquelle ladite conduite de refoulement et ladite conduite d'aspiration sont formées à travers un tube allongé d'un appareil endoscopique, et ledit cylindre de soupape est disposé sur une poignée de commande dudit appareil endoscopique.

3. Unité de soupape de commutation selon la revendication 1, comprenant en outre :
un premier dispositif antirotation pour réguler une rotation relative entre ladite tige de piston et ledit dispositif de bouchon, et
un second dispositif antirotation pour réguler une rotation relative entre ledit dispositif de bouchon et ledit cylindre de soupape.

4. Unité de soupape de commutation selon l'une quelconque des revendications 1 à 3, dans laquelle ladite chambre de piston inclut une surface à diminution progressive disposée avec une inclinaison vers ladite ouverture de réception, et
ledit trou d'orifice d'aspiration est disposé d'une manière observable en externe à partir de ladite surface à diminution progressive.

5. Unité de soupape de commutation selon la revendication 3 ou 4, dans laquelle ledit second dispositif antirotation inclut :
une saillie de prise formée sur un premier élément parmi ladite surface à diminution progressive et ledit dispositif de bouchon ;
un retrait de prise, formé dans un second élément parmi ladite surface à diminution progressive et ledit dispositif de bouchon, pour recevoir ladite saillie de prise en vue d'une prise.

6. Unité de soupape de commutation selon la revendication 4, dans laquelle ladite tige de piston inclut une surface de trajet d'écoulement, formée dans une surface périphérique plus longue qu'une course entre lesdites première et seconde positions dans ladite direction axiale, présentant une portion de positionnement dudit trou d'orifice d'échappement tout en étant positionnée dans lesdites première et seconde positions, en vue d'un alignement avec un espace dans ladite surface à diminution progressive tout en étant positionnée dans ladite première position, pour constituer ledit premier passage.

7. Unité de soupape de commutation selon l'une quelconque des revendications 1 à 6, dans laquelle ladite tige de piston inclut :
une portion de grand diamètre disposée près de ladite extrémité supérieure de ladite chambre de piston ;
une portion de petit diamètre, formée sous ladite portion de grand diamètre, présentant un diamètre plus petit que ladite portion de plus grand diamètre, et présentant ladite garniture d'étanchéité, pour permettre à un fluide de s'écouler à l'intérieur de ladite chambre de piston ;
ladite chambre de piston inclut :
une surface de coulissement de piston pour guider ladite portion de grand diamètre d'une manière permettant le coulissement ;
une surface d'étanchéité de coulissement, disposée sous ladite surface de coulissement de piston, pour maintenir ladite garniture d'étanchéité d'une manière permettant le coulissement et l'étanchéité aux fluides.

8. Unité de soupape de commutation selon la revendication 7, dans laquelle ledit troisième passage inclut :
une première surface de trajet d'écoulement, formée dans une surface périphérique de ladite portion de grand diamètre pour s'étendre dans ladite direction axiale, et alignée avec ledit trou d'orifice d'échappement ;
une seconde surface de trajet d'écoulement, formée dans ladite surface périphérique de ladite portion de grand diamètre suivant un angle de phase différent de ladite première surface de trajet d'écoulement, pour correspondre avec une portion inférieure de ladite première surface de trajet d'écoulement par référence à ladite direction axiale, et opposée à ladite surface de coulissement de piston ;
un trou de passage, formé à travers ladite portion de grand diamètre, pour s'ouvrir à travers chacune desdites première et seconde surfaces de trajet d'écoulement, et
dans le cas où ladite tige de piston se trouve dans ladite seconde position, ladite première surface de trajet d'écoulement, ledit trou de passage et ladite seconde surface de trajet d'écoulement ouvrent un trajet d'écoulement entre ledit trou d'orifice d'échappement et ledit passage de surpression.

9. Unité de soupape de commutation selon l'une quelconque des revendications 2 à 8, dans laquelle ledit appareil endoscopique inclut en outre un ballonnet, disposé sur un dispositif de bout dudit tube allongé, relié à une extrémité de ladite conduite de refoulement, en vue d'un ancrage dans ladite cavité corporelle.

10. Appareil endoscopique incluant une poignée de commande (12), un tube allongé (11), disposé pour s'étendre à partir de ladite poignée de commande longitudinalement, pour imager un objet dans une cavité corporelle, et un ballonnet (21), disposé sur un dispositif de bout dudit tube allongé, en vue d'un ancrage dans ladite cavité corporelle, ledit appareil endoscopique comprenant :
une conduite de refoulement (35), formée à travers ledit tube allongé, pour un raccordement audit ballonnet ;
une conduite d'aspiration (28), formée à travers ledit tube allongé et présentant une ouverture d'aspiration positionnée au niveau dudit dispositif de bout dudit tube allongé ;
une conduite d'échappement (46), disposée pour s'étendre de ladite poignée de commande vers un côté proximal, pour un raccordement à une source d'aspiration (45), et
une unité de soupape de commutation selon l'une quelconque des revendications 1 à 9,
dans lequel ledit trou d'orifice de refoulement (56) communique avec ladite conduite de refoulement (35) ;
ledit trou d'orifice d'aspiration (57) communique avec ladite conduite d'aspiration (28), et
ledit trou d'orifice d'échappement (58) communique avec ladite conduite d'échappement (46).

11. Unité de soupape de commutation selon l'une quelconque des revendications précédentes, comprenant en outre un bouchon à bouton (47) en vue d'une manipulation monté de manière fixe sur un bout de la tige de piston (51).
